# EUROPEAN PATENT SPECIFICATION

(11) **EP 3 322 476 B1**
(45) Date of publication and mention of the grant of the patent: **21.11.2018**
(21) Application number: 16757934.1
(22) Date of filing: 23.06.2016
(51) Int. Cl.: A61N 1/20, A61N 1/05

(54) **DEVICE FOR DISINFECTION BY MEANS OF IONIC CURRENTS**
VORRICHTUNG ZUR DESINFEKTION MITTELS IONISCHER STRÖME
DISPOSITIF POUR DÉSINFECTION AU MOYEN DE COURANTS IONIQUES

(30) Priority: 13.07.2015 IT UB20152119
(43) Date of publication of application: 23.05.2018
(73) Proprietor: Lagarde, Philippe Edouard Joseph Paul, 47891 Falciano (SM); Lagarde Pajot, Josiane, 47891 Falciano (SM)
(72) Inventor: CASOLI, Lino, 21010 Agra (Varese) (IT); LAGARDE, Philippe Edouard Joseph Paul, 32380 Avezan (FR)
(74) Representative: Pasquino, Fabio
(86) International application number: PCT/IB2016/053742
(87) International publication number: WO 2017/009727

(56) References cited:
- EP-A2- 1 134 002
- FR-A- 916 843
- US-A- 2 276 623
- US-A1- 2004 101 809

## Description

### Field of the invention

The present invention refers to a device of sterilization based on the ionic current, for sterilizing the ducts of the teeth that are infected by pathogen agents.

### Background art

The principle of treating infected teeth is already known in the specialist literature as well as from a plurality of patent documents, among which we cite, only as an example and with a non-limiting extent, the following:
DE 27 28 494 A, DE 28 06 297 A, US 2 276 623 A, EP 1 134 002 (the applicant of this last document corresponds to the applicant of the present patent application).

In DE 27 28 494 A is described a system constituted by a galvanic element, pin shaped, suitable for performing a long lasting ionic current. The system does not require the application of an external current source. The pin is constituted by a part in zinc of a spherical shape (first electrode) and by a needle in copper adequately treated (second electrode) and that is inserted in the tooth channel. In the point of transition between the first and the second electrode realized in a single section with the first one, there is a sort of insulating cuff for avoiding current dispersions. The system is totally different from the system adopted in the present patent application for the following reasons:
1) in the tooth a solution (aqueous paste) with Calcium hydroxide (Ca(OH)2) and Copper hydroxide (Cu(OH)2) is introduced;
2) it is not possible to precisely control the total amount of charge that passes from one electrode to the other into the oral cavity from the beginning up to the end of the treatment;
3) the system rests in the mouth for a significant amount of time, while it could be envisageable that the intervention resolves in a few minutes or at least about in a quarter of hour;
4) the system requires the forming of a cavity/seat for housing the zinc sphere (first electrode) with the associated cement and therefore the intervention is more invasive;
5) the system does not require any external instrument;
6) there is no direct verification of the fact that the treatment had the desired effect (if for example the insulation is not perfect, there are dispersions of currents which are not controllable).

The document DE 28 06 297 A describes instead a method of dental treatment of the periodontosis that involves an electrode containing silver ( or an electrode in cloth soaked in silver) to be introduced in the gingival pocket. The system is connected to an external source of constant direct current. The silver ions produce the disinfecting effect. This method relates to a therapy to apply for about 10 minutes (see page 6, third paragraph, of DE 28 06 297 A). This period of time, incidentally completely indicative, is advised for a pathology (periodontosis) and for a method of antibacterial treatment (introduction of the electrode laterally respective to the tooth and not in the channel of the tooth) that are completely different from the scopes of the present invention. Furthermore, silver ions instead of OH- ions are used for disinfection.

The document US 2 276 623 A uses needles that act like electrodes and are introduced in the channels of the tooth pushing therein a solution of iodine. The needles are made advance in the channel up to a predefined distance from the apical hole. The current should rest constant, but in truth there is no control on the real value of the current, neither of the time and therefore on the electric charge. Furthermore, there is no description of means for insulating electrically in an appropriate way the needles (negative electrodes), so there is neither control of the current dispersion and then on the effectiveness of the therapy. In effect, the electrical isolation is made for the wire (11) that conduces to the negative electrodes, using small long tubes (15), but not between the lateral wall of the needles (17) and the upper wall of the tooth channel ( that is instead of a fundamental importance for avoiding current dispersions like it will be described hereinafter). The positive electrode, constituted by two circular plates arranged in contact respectively with the external and internal side (oral cavity) of the cheek of the patient, renders furthermore the treatment is only slightly comfortable for the patient.

The document EP 1 134 002 describes a sophisticated system for performing disinfection of ducts (tooth channels) and of dental tubules. Thanks to this document, that for the first time poses in foreground the importance of the availability of a device suitable for precisely dosing the amount of electric charge delivered (measured in Coulomb). Said device therefore has a voltage generator and a device for keeping constant the current in the circuit, together with an associated timer. The device produces it antibacterial function by means of a passage of OH- ions through the dentinal tubules. Anyway, in this document is not described:
a) a suitable electric insulation of the upper part of the needle-shaped electrode respective to the upper proximal part of the tooth's radicular channel, and this leads to dispersions of current in a transversal direction, for which there is no certainty that the delivered current passes entirely through the aforesaid dentinal tubules. In other words, also keeping constant the current in the circuit, as it is described by the device according to patent application EP 1 134 002, there is no full certainty that the delivered current, once initially set (and one single time for the duration of the whole treatment) by the operator, multiplied by the time of treatment set by the operator (and controlled by the timer), actually corresponds to the overall electric charge that actually has traversed the dentinal tubules and that should correspond to the nomograms values (empirically defined), to the end of guarantying the effectiveness of the therapy.
b) on which values of current (of the delivered current), and over what period of time of current passage in the dentinal tubules, the therapy shall be based, in case of canine teeth, molars and premolars. In other words, the software indicated in EP 1 134 002 (even if distinguishing between the various pathologies) does not provide for a distinction between the various types of teeth, precise data to adopt for the various types of teeth not being provided, thereby controlling only current and timer.

Actually, the problem is mentioned, and it is said that the position of the tooth in the dental arch shall be kept into account, but it is not managed so as to be technically solved.

Neither there is confirmation of the effectiveness of the therapy, once the treatment is completed, since there is no provision of a sound that determinates the alkalinity value produced at the tooth base by the OH- ions ( in correspondence of the exits of the dentinal tubules). Therefore, also supposing of providing an electrical insulation into the high part of the needle (electrode), as it is provided actually only by the present invention, since the dispersions could take place also in another mode (see below), there is no confirmation of the actual passage of a precise amount of electric charge.

It would be envisageable that a device is capable of measuring the pH - or another parameter - indicative of the conditions of the tooth so as to set the intensity of the current delivered in the course of the treatment. During the activity of tooth's treatment, it is useful to precisely monitor the presence of OH- ions on a particular zone of the tooth that is treated so that the sterilization can be performed with the maximum possible precision without intervening on the surrounding parts.

There is knowledge of sensors that offer specific functionalities for example for measurement of ph. Some sounds are provided for detecting the depth of the decay of the gingiva, they comprise a needle and a handle wherein the needle has tapered graduations so as to let the measurement be possible. The needle point can be shaped for particular points of the decay.

Said sound can be easily adapted for measuring pH levels, to the measure of temperatures and other parameters. Since the dentist can accurately drive the tip of the sound needle in a particular area of the tooth, an accurate and direct measurement of the pH level can be obtained with a sensor located in correspondence of the tip of the needle itself. The dentist can also accurately measure the temperature with a temperature sensor on the tip of the needle.

It is then useful to premise again a brief introduction on the structure of the tooth in relation to measurable pH levels. The tooth, integrated into a mascellar bone, covered by the gingiva, has therein a pulp inserted into the dentin (the part of ivory) and in the enamel. The enamel is basically constituted of a mineral whose the main component is the hydroxyapatite. The dentin is composed at 65% of a mineral, at 18% of organic material and at 12% of water. As already indicated, there are also tubular structures constituted in the dentin that have an important role when a decay is in development and bacteria attack the tooth. These tubular sections acquire his information by immediately detecting that the tooth has been attacked and then new and stronger tissues are created for blocking the invasion of bacteria. With reference to the formation of the decay, some studies have proposed that microorganisms into the plaque generate a series of types of acids that in turn produce decay. The decay pH has been many times experimentally measured: the patient with a minimum number of decays has been found to have a plaque with a pH comprised between 5.2 and 6.7 whereas the patient with many decays has a plaque characterized by a pH comprised between 4.0 and 5.2. To the end of actuating an appropriate and complete treatment of the decay on the basis of objective parameters, studies based on the pH values have been carried out with the scope of clinical treating the decay itself. The more recurring results refer to the exam of a coagulated film with pH values in the order of 6.8 up to 7.3 for zones of dentine in a good state; on the other hand, for an acute decay of the tooth with dolorous decays that do not allow the person to sleep at night, there was a definition of a coagulated film characterized by a pH comprised between 5.4 and 5.8. For a chronic dental decay, the minimum detected pH for the film of gel was comprised between 6.3 and 6.6.

From the point of view of the acute and chronic decays have been defined the relative ranges of values for identifying if the decay can be treated or can, up to a certain degree, be left as it is (cfr, studies of Dott Muramaki Keiko and Kitasako Yuichi of the Tokio Medical and Dental University).

In the cited studies, it has been noticed the possibility of using a pH image detection system so that to examine the activity of the decay. The decay was red colored using a non-acid red solution, and it was possible to clearly distinguish the area of the tooth in good conditions from the decay to submit to treatment. In the same tooth it has been found a general lo pH in the center of the decay, with a pH that rises to a higher value (if compared to the decay's one) when moving towards the section of the tooth in a good state.

Therefore it has been developed a pH micro sensor suitable of evaluating the surface pH, punctual, for dentine with stopped or active decays. In particular have been made studies on the use of the techniques relating to the pH image and for all of these techniques, the chronic or acute decay's pH values have been statistically reported in relation to the pH values of an healthy dentine. With all the various techniques of detection, relevant coincidences of the values of pH inherent to active and stopped decays have been found.

The fundamental principle is that the pH image sensors are a new technique of potentiometric detection, known as LAPS (Light Addressable Potentiometric Sensor, potentiometric sensor with addressable light). In said sensor, layers of isolating film are laid on a silica substrate, and then the sample, an electrolyte, is arranged over the insulating film. When the light is directed on the opposite (non-covered) side of the silica substrate, a photonic current is generated, and this corresponds to the sample's pH. By changing the position of the light source, an image relating to the distribution of pH in the sample can be obtained. For understanding in detail as the pH is measured, we shall keep into account that when outside the sensor a negative polarization is applied, the effects of the electric filed are so that the electrons of the semiconductor move, thereby creating depletion layers, areas wherein there are no electrons (for n-type semiconductors), where the sensor develops a capacitance. Then, when the light is applied in an intermittent way, there is an electrostatic capacitance generated by the effect of the AC photocurrent. Recently alternative solutions to most relevant problems have been provided, and these are the speed of measurement, the accuracy of pH measurement and the space resolution. It shall be furthermore noted that this type of device is suitable for performing real-time dentine pH measurements, and in fact today it is provided a use with portable devices to be provided to the user that can immediately evaluate the status of his tooth and can in real time intervene on the tooth with a drug or other substance, modifying the pH thereof.

The applicant has furthermore noticed that one of the issues that are found by doctors during the therapy of an ill tooth, resides in the need of a manual programming of a complex electronic device. Many times, in fact, the practitioner faces the need of urgently intervene on a patient by using an electronic device with very technical voltage or current commands.

The applicant has further noticed that in case the current passes in a portion neurologically irradiated, the current passage shall be guided on a specific path, avoiding spurious current leakages that - in the path between two electrodes - could pass in non-correctly de-sensitized or anesthetized points of the body, causing pain and anyway reducing the effectiveness of the therapy.

The scope of the present invention is therefore to describe a device for bacterial disinfection using ionic current, which allows for solving the aforementioned drawbacks.

The scope of the present invention is furthermore to describe an electrode for said device, that allows to solve the aforementioned drawbacks.

### Summary of the invention

According to the present invention is realized a device for disinfecting by means of ionic current, said device being characterized in that it comprises a principal body provided with of a data processing unit suitable for feeding a constant current generator with a signal of generation of a electric current fed to a couple of electrodes electrically correlated thereto, and comprising furthermore a user interface device configured for allowing the selection of a determined pathology between a plurality of pathologies in advance stored within a memory electrically connected with said data processing unit, wherein for each pathology of the said plurality of pathologies is in advance stored in said memory a interval of values of electric current and/or of electric charge and/or a specific value of electric current and/or of electric charge that said constant current generator shall deliver during said therapy, and wherein said couple of electrodes comprises a first electrode of an elongated type suitable for being introduced or anyway laid in proximity of an infected or inflamed portion of human body, said portion being a bone or tooth or their respective cavities, and a second electrode suitable for being grasped by an ill patient.

Advantageously, within said memory are stored, for each pathology of the said plurality of pathology, a first interval of values of electric current and a second interval of values of electric current and at least a first value of charge; said first interval and said second interval being alternatively selected by said data processing unit in accordance to a selection command imposed by the user to said data processing unit.

Advantageously, said user interface means are configured for allowing the selection of one between a plurality of patient sensitivity levels and for causing the transmission of said signal of generation of electric current to said constant current generator in accordance to said selection performed by said user, said transmission of said signal of generation of electric current being performed in relation to said selection command.

Advantageously, said device comprises sensor means of current, measuring the electric current flowing between said first and second electrodes said sensor means of current being interfaced with said data processing unit for calculating an electric resistance to the passage of said current and for interrupting said passage of electric current between said first and second electrode in case said electric resistance exceeds a threshold value in advance stored within said memory and/or said value of electric resistance mutates with a derivative exceeding a predetermined value; said data processing unit calculating continuously said value of electric resistance. According to a preferred and non-limiting aspect of the present invention, said interval of values of electric current is comprised between 0,5mA and 5mA, and said value of charge is comprised between 100mC and 1000mC; and wherein for each pathology selected between said plurality of pathologies in advance stored said signal of generation of electric current and/or charge requires an electric current and/or charge comprised in the interval associated to said selected pathology.

Advantageously, said first electrode comprises a needle-shaped portion ending in an extremity thereof with a tapered pointed portion; said first electrode furthermore comprising an electrically insulated section covered by an electrically insulating layer of material or paint of thickness lower than the section of said needle-shaped portion. Advantageously the insulation takes place by means of a paint specifically conceived for enveloping highly bent surfaces with a low diameter.

Advantageously, said first electrode comprises a needle-shaped portion and an extremity thereof with a rounded point with size greater respective to the section of said needle-shaped portion; said first electrode comprising furthermore an electrically insulated section, covered by a layer of electrical insulating layer of material or paint of a thickness lower than the section of said needle-shaped portion. Advantageously, said electrode alternatively comprises a needle-shaped portion ending in a first extremity thereof with a double point suitable for introducing in a plurality of channels of said tooth.

Advantageously the first end can have more than two points. This is advantageously useful in case there are teeth characterized by a great plurality of channels.

In detail, said user interface device is configured for allowing to select a specific type of bone or tooth between a plurality of types of bone or tooth, and wherein said electric current and/or charge delivered from said constant current generator towards said couple of electrodes is delivered in accordance to a signal of generation of an electric current being transmitted from said data processing unit towards said constant current generator in accordance to said specific selected type of bone or tooth.

In detail, said type of tooth is selected in accordance to a number of channels of said tooth, and wherein said data processing unit calculates a predetermined time of transmission of the said electric current to said couple of electrodes for each channel of the selected type of teeth, and is configured for performing a cycle of sending of an alert message on said user interface means in case the predetermined time for said therapy of said channel is ended, wherein said cycle is performed for a number of times equal to the number of channels of said tooth minus 1; said data processing unit being configured waiting for a command of the user for sending the signal of generation of electric current for curing the adjacent dental channel, up to the moment when the cycle is reset.

According to a preferred aspect of the present invention, the device herein described is specifically conceived for dental disinfection.

According to a preferred aspect of the present invention, the device herein described is specifically conceived for the disinfection of metal parts of the body.

According to the present invention it is furthermore described a method of sterilization of objects by means of ionic current, said method being characterized in that it comprises a step of positioning of a first and a second electrode on electrically conductive portions of said object, and comprising furthermore a step of delivering of a electric direct current by a device for disinfecting by means of ionic current electrically connected a said first and second electrode and providing said electric current to said first and second electrode; and wherein said electric current to be delivered to said electrodes is selected between a plurality of values in advance stored in a memory of said device and, when delivered, causes a generation within or in proximity of a humid portion of said object, of OH- ions realizing said sterilization.

Advantageously, said method comprises a step of manual preselection of an amount of electric charge to be delivered to said first and second electrode; said selected amount of electric charge increasing with the increase of the bacterial charge which is supposed or verified on sand object, and wherein said device delivers said first and second electrode a constant electric current, varying the time of delivering in direct accordance with said amount of preselected electric charge.

In detail, said object is a metal prosthesis suitable for being inserted within a portion of human body, and wherein said method comprises a step of wetting said metal prosthesis before delivering said electric current.

Alternatively, said object is an electrically conductive food container, filled of food in an at least partially wet configuration, and wherein a first electrode between said first and said second electrode is arranged directly on said container and a second electrode between said first and said second electrode is positioned in direct contact with said portion at least partially humid portion of said food.

Advantageously, finally, the method comprises a step of continuous electronic verification of an amount of electric resistance between said first and said second electrode during the delivering of said direct electric current, said step of electronic verification being performed by a data processing unit of said device and comprising a step of interruption of said delivering of electric current to said first and second electrode in case said value of electric resistance or a temporal derivative thereof increase over a predetermined level.

### Brief description of figures

The present invention will be now described referring to a particular and non-limiting embodiment, shown in the annexed figures, wherein:
figure 1 shows a basic block scheme of the structure of the device object of the present invention;
figure 2 shows a detail of an electrode of the device object of the present invention;
figure 3 shows circuit block scheme of the device object of the present invention;
figure 4 shows a flow chart of the operation of the device object of the present invention;
figure 5 shows a first alternative embodiment of a point of said first electrode;
figure 6 shows a second alternative embodiment of a point of said first electrode
figure 7 shows a time-pH diagram;
figure 8 shows a second time-pH diagram;
figure 9 shows a plurality of points of radicular infection of teeth.

### Detailed description of the invention

In the procedure of sterilization of the radicular channels and dentinal tubules based on the passage of OH- ions for obtaining this antiseptic effect, there are the following advantages:
- the device is of an external power source type, and therefore does not create discomfort to the patient since no one of its components rests in the patient's mouth, the treatment being performed by the dentist in the arch of some minutes and for a maximum of about a quarter of hour;
- the negative electrode is isolated from the tooth by the upper part of the channel, while the remaining portion (lower) rests into the cavity of the channel (or free space), thereby guarantying a significantly higher effectiveness level since the risk of dispersion of currents reduces;
- the device can be set by the operator in accordance to the type of infection / pathology, as well as according to the position of the tooth in the dental arch,
- the device operates in a precise and efficient way, guarantying the correct electric charge of traversing the dentinal tubules, in any case basing on the correct electric isolation and of the electronic system against the time and of the current (constant), that are values set by the operator, which cannot fall in error since will only have to push buttons for each specific case, and then will be the system to automatically choose the current and time values (through the internal associated table of consultation);
- the device has a pH sound (for example needle-shaped) that performs a monitoring of the degree of alkalinity (OH- ions that have transited the dentinal tubules and the radicular channel): these data are communicated in real time to a control unit (microprocessor) that can stop the operation of the device (and therefore the treatment) in case of values too different than the nominal ones, furthermore starting a monitoring with the control of a closed loop integrated in the device that realizes the process of sterilization using the ionic current;
- preferably there is a display that reports the levels of monitoring detected by the pH sound. There is therefore a confirmation, also visual, that the treatment correctly takes place.

In case of significant difference respective to the value of the nominal charge (desired electric charge), the negative electrode is substituted (which is of a disposable type) with another, and it is ensured that the new electrode is introduced in the correct/precise position and is not defective (wrong electric insulation). The treatment can therefore be immediately repeated. Anyway, this eventuality shall be considered significantly not probable.The present invention bases, as aforementioned, on a deep study on the problem of sterilization of an infected tooth, that for having a positive result shall guarantee the satisfaction of a plurality of conditions (requisites). Furthermore, once all the necessary parameters/variables are acquired, basing on a detailed research, it was necessary to put into practice these results realizing the most possible efficient/precise system.

First it has been possible to find that was imperative to isolate the negative electrode from any contact in the zone upstream the real and actual channel wherein the electrode was inserted.

No metal, cloth, object, humidity (saliva) should come into contact with the electrode. If this first condition was not satisfied, the dispersion of current led to a significant decrease of the production of OH- ions in the desired point, that is in the tooth's principal channels but also in the secondary ones, without forgetting the dentinal tubules, of which no one has ever taught if not in the last years.

This means that the negative electrode insulation is extremely important.

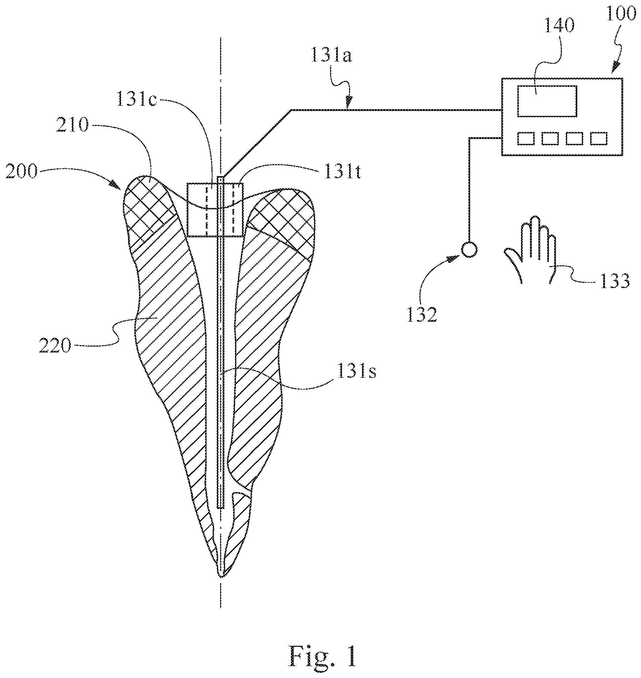

The negative electrode of the present invention constitutes an electrode of a disposable type, comprising an insulating "cap" 1 in wax, cork, rubber, or other material preferably not expensive, according to the annexed figure. It is therefore possible to use the electrode also through a metal (crown) 210 (Fig. 1), if the conditions require so, and this was not possible in the systems of sterilization with OH- ions of the known technique.

In detail, from the device 100 object of the present invention, as represented in figure 1, start a plurality of electrodes used for curing the tooth or a bone infection. In detail, concerning the dimensional ratio between the tooth 200 and the device object of the present invention, the measures are not in scale.

In detail, in the course of the present description, reference will be made explicitly to the cure of the tooth channel.

In detail, said device 100 comprises a couple of electrodes 131, 132 configured for sending a current through the body of a human being.

The first electrode 131 of the said couple of electrodes is shaped in such a way to be capable to insert within the dental channel; the second electrode 132, instead, is configured for receiving the signal of current that flows from the first electrode 131 towards the hand of the patient.

As it is schematically shown in figure 1, the first electrode 131 introduces within the radicular channel 220c of the tooth 200, whose upper portion presents a crown 210. In detail, a needle-shaped portion 131s extends ideally up to the apical point of the radicular channel.

The first electrode 131 has above a cap 131t, that allows of insulating the radicular channel form the rest of the environment. The cap 131t has a section with a size significantly greater respective to the section of the needle-shaped body 131s of the tooth, so that to be capable to block itself into the mesial upper portion of the radicular channel.

Into the cap 131t extends a plurality of channels 131c that allow the passage of gases produced by the ionic current outside the radicular channel; this advantageously allows for reducing the risk of damaging the tooth due to an overpressure that creates into the radicular channel itself.

As it will be better described in detail, the electric current is made flow between the first electrode 131 and the second electrode 132, realizing a production of OH- ions that allows for sterilizing the infected radicular channel and the immediately surrounding gingival portions. Within the tooth, and in detail in the radicular channel, thanks to the effect of the ionic current, H+ ions are created, that in the humid peri-apical environment produce an electrolytic foam.

Said electrolytic foam shall be regularly dried for avoiding that the humidity produces a current derivation coming into contact with the surrounding environment. For said reason, the device 100 object of the present invention is optionally provided with a pH sound, suitable for detecting the presence of an excessive concentration of electrolytic foam.

If said foam shall not be regularly dried by the dentist, or if this operation does not take place correctly, the dispersion would be signaled by the sound p, and the dentist should intervene for performing the operation of foam drying (this could take place if the dentist, temporarily being in another room during the treatment, forgets to do it). Anyway, it can be seen that the pH sound constitutes a further safety device for the correct development of the disinfection operation.

Preferably, the cap 131t is realized in a not expensive material such as for example and in a non-limiting extent wax, cork, rubber or other similar material.

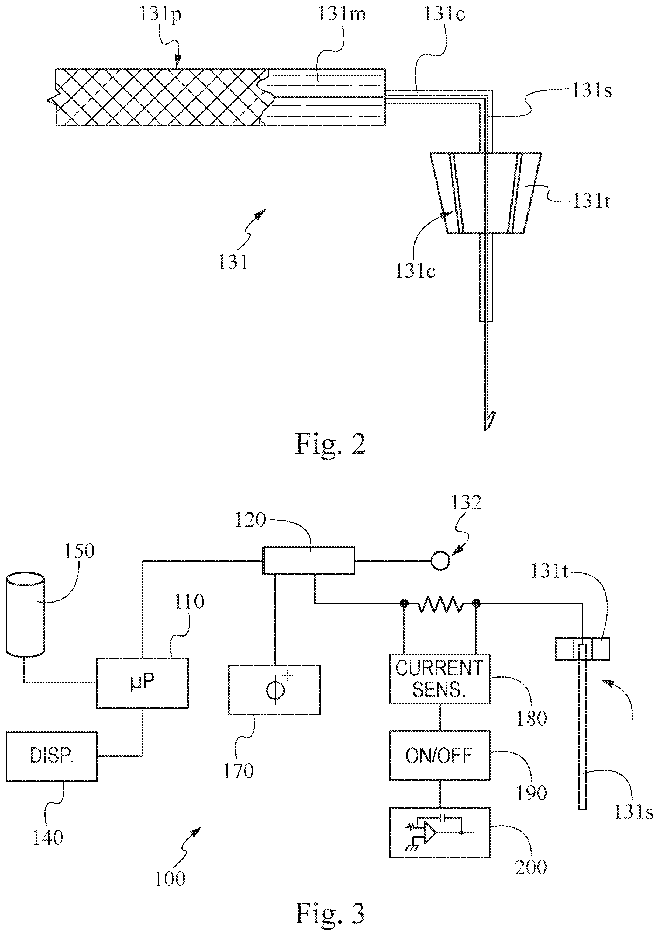

As it is shown in figure 2, in a first form of realization the electrode 131 that shall be in use introduced in the channel of the tooth 200 of the patient, comprises a needle-shaped electro-conductive body 131s introduced in an electrically insulating cap 131t.
the electrically insulating cap 131t, has a first central hole conceived for allowing the passage of said needle-shaped electro-conductive body; the electrically insulating cap comprises furthermore a plurality of holes 131c passing between an its first lower surface and an its second upper surface that allow for the passage of gases that develop during the therapy.

The upper portion of said electro-conductive body 131t, and that is the portion more in proximity of the cap, is covered by a layer or film of electrically insulating material. Therefore, observing the area of the electro-conductive body 131t that is under said cap, it is noticed a first electrically insulated zone and a second electrically conductive zone.

In a preferred and non-limiting form of realization of the present invention, the first electrically insulated zone of the electro-conductive body is insulated by means of a special paint. In fact, said paint is specifically conceived for covering elements of small diameter, characterized by a strong bend (if clearly observed), that would make a stable covering difficult or even impossible.

Said second zone is the one that in use extends more deeply in the radicular channel of the tooth.

Said insulation is useful for reducing losses of current that would disperse because of humidity and saliva present into the upper portion of the tooth without becoming part of the channel therapy.

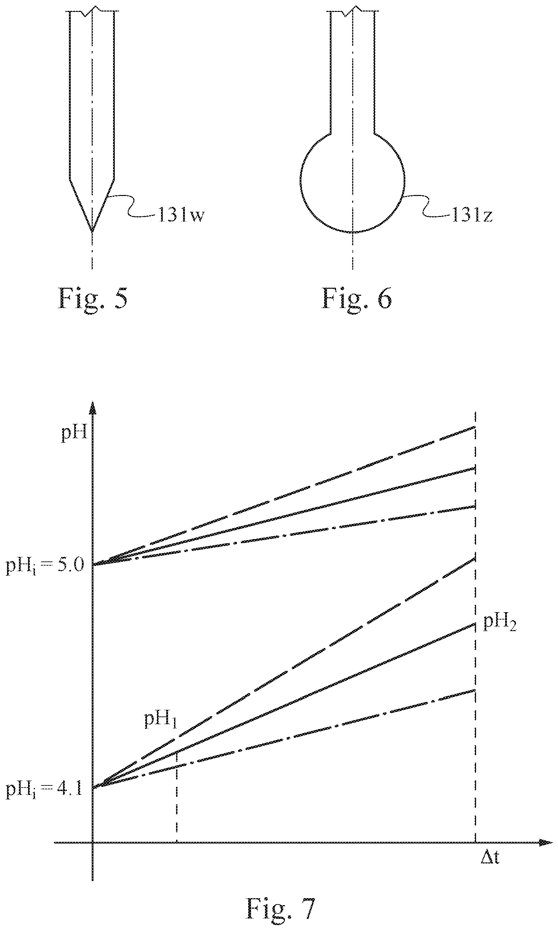

As it is shown in figure 5, in a first alternative version, the ending portion of said needle-shaped electro-conductive body 131 is tip-shaped 131w, that closes in correspondence of an axis of said needle-shaped electro-conductive body 131.

The needle-shaped electro-conductive body, upperly at cap 131t, is curved with an angle preferably of 90° and introduces within a grip or handle 131p electrically insulated respectively from said electro-conductive body 131t. The insulation of the grip or handle is important for avoiding unwanted secondary electro-conductive circuits between the hand of the doctor and the hand of the patient who handles the second electrode 132.

Preferably said grip or handle 131p are realized in metallic material, and in detail in surgical inox steel. The needle-shaped electro-conductive body is introduced in a hole of said grip or handle; said hole is covered by an electrically insulating layer 131m.

On the rear side, from said grip or handle, departs an electric cable connected to the constant current generator that will be better described in the subsequent part of the description.

In a second form of embodiment, shown in figure 6, said electrode 131 differs from the one shown into the first form of embodiment because an its ending portion is sphere-shaped 131z, of which the diameter is higher with respect to the thickness of the rest of said needle-shaped electro-conductive body 131t.

This advantageously allows for having a more uniform current distribution in the channel, both at zenith level and at azimuth level. Substantially, therefore, in the deepest point of the channel there is a better current distribution, that in particular in big sized channels contributes to a better effectiveness of the therapy.

A third embodiment of the electrode in the system object of the present invention is characterized in that said angle is an angle lower than 90° and preferably owner than 60°.

A fourth embodiment of the first electrode 131, not shown, advantageously comprises a terminal portion of said needle-shaped electro conductive body 131t shaped with a plurality of points arranged preferably but in a non-limiting extent as a fork. This type of electrode is particularly advantageous when the therapy of sterilization takes place on a pluri-radiculated tooth, that is provided with a plurality of radicular channels.

A fifth embodiment of the first electrode 131, not show, does not comprise a needle-shaped body, but an electro conductive body shaped as a platelet. Said body is particularly useful when the infected portion of the radicular channel is the upper mesial of the tooth, characterized by a diameter greater respective to the apical portion of the radicular channel. Said bent platelet shaped body allows for a significantly more uniform, and therefore more effective distribution of current density on the portion to be sterilized.

All the five embodiments of the electrode described in the present invention can be provided with an activation or deactivation pushbutton for the passage of electric current. Said pushbutton is conveniently positioned in correspondence of the handle or grip 131p, and is covered by a material with hydraulic sealing, so that eventual humidity or fluids present on the hands of the dentist do not interfere with the interruption of the flow of electric current in the electro conductive body 131t. Said pushbutton controls a switch positioned within the handle itself.

According to a characterizing aspect of the present invention, the control of a plurality of parameters of operation of the device 100 is managed automatically by a control unit, so that to avoid that the practitioner shall manually selected the intensity of current or charge which shall applied in accordance to the gravity of the dental pathology treated.

In detail, as it is shown in figure 3, the device 100 object of the present invention comprises a microprocessor 110 or data processing unit, electrically connected with a user interface device 140 preferably but in a non-limiting extent realized by means of a touch-sensitive display, that allows both the visualization of menus generated by the data processing unit as well as receiving commands that are set by the user or practitioner and transmitted to the data processing unit for a subsequent processing. The data processing unit is furthermore connected also with a memory 150. Within said memory is in advance stored a table, that is hereinafter shown.

| Pathology | Charge (mC) | current (mA) | current(mA) |
|---|---|---|---|
| | | Normal patient | Sensitive patient |
| Healthy pulp | 400 | 2,5÷3 | 1,5÷2 |
| Pulp necrosis | 500 | 3 | 2÷2,5 |
| Purulent | 500 | 3 | 2÷2,5 |
| Loco-regional | 600÷800 | 3 | 2÷2,5 |

In phase of design and study of the system object of the present invention, several studies have brought to the definition of standardized parameters typical for each type of pathology.

As it is shown into the above table, within the memory associated to the data processing unit, there are a plurality of default values for four levels of increasing seriousness pathology. In detail:
- for a first level of pathology, the dental pulp is substantially healthy but has an hyperemia causing the pulpitis.
- for a second level of pathology, it deals with necrosis of the pulp;
- for a third level of pathology, it deals with purulent pulp inflammation
- for a fourth level of pathology, it deals with a loco-regional inflammation.

In a first column of said table there is plurality of pathologies which are typical of the radicular channel of the tooth. In detail, in the proceeding with the increase of row on the same column, the pathology is increasingly more serious.

In a second column of said table there are the values of charge, expressed in Coulomb, which are univocally associated to said plurality of pathologies.

In a third and fourth column of said table, there are values of electric current that shall be made to flow between said first and second electrode 131, 132 during the therapy of the damaged tooth.

Therefore, for each type of pathology, there is a tern of intervals of values, of which a first of electric charge to deliver, and a second and a third of electric current to deliver.

The third and fourth column are characterized by being associated with values of electric current to respectively deliver to sensitive or normal patients. The values of electric current to deliver to normal patients exceed the values of electric current to deliver to sensitive patients.

The association between the electric charge and the electric current to deliver to the couple of electrodes 131, 132 allows for defining also the time of duration of the therapeutic intervention, since the electric charge is the product of the electric current per unit of time.

This temporal calculation element cannot be disconsidered if a favorable and reproducible result is required. It would be preferable to know the exact pathology, i.e. the degree of infection, the entity of its extension, and finally, if possible, also the importance of the nature of the germ or germs responsible thereof.

The sperimentation in vitro for obtaining the necessary table of values which is above recalled, to be used as a basis for the software that is executed by the data processing unit, took place as follows.

An infected tooth (extracted) has been immersed with its radicules in a solution containing NaCI (sodium chloride) gel, and fenoftaleine. The positive electrode has been partially immersed into the liquid solution, the negative electrode has been introduced (with its insulating cap) in the principal channel of the tooth up to a depth of 2/3rds of its length. Then an electric current at a predetermined intensity has been made pass. After a certain amount of time, a subsequent appearance of "red color reactions" can be verified in correspondence of the holes of the principal channel, then of the secondary channels and then on the entire surface of the radix immersed in the solution. The number indicates a OH- ion that exited from a secondary channel or dentinal tubule.

Said red color reactions are caused by the alkalinity of OH- ions, that after having transited through the channels, diffuse into the solution containing fenoftaleine. This experimentation, up to this precise stage, was substantially yet developed by Pierre Bernard, and does not constitute a novelty. The actual important result for the purposes of the present patent application, is the following of this experimentation. Actually, Pierre Bernard did not notice that according to the diameter of the channel which is travelled by OH- ions, the appearance of the reaction with the fenoftaleine was more or less rapid. Actually he did not realize also that after a certain amount of time the entire surface of the radix of the tooth became red colored.

All of this has found a response recurring by an electronic microscope, as it has been made by the inventor of the present patent application, and basing on the presence of microscopic tubules in the dentine. Actually the passage inside the dentine tubules is slow and can be observed only at the end of the experimentation. Simultaneously, this explains as the technique of the present invention is precise and suitable for sterilizing the tooth in a fully reliable way.

Subsequently (always in vitro) have been performed repeated bacteriologic analyses, on the channels of the teeth which have been treated with various levels of electric charge and speed of passage (intensity of current). This has allowed of stabilizing progressively a set of values of application of the technique object of the invention. Considering the aforementioned experimentation, according to the present invention, it has been considered of suing a pH sound for example needle-shaped (for measuring the alkalinity produced by OH- ions in proximity of tooth radix) that communicates the so obtained detection data to a microprocessor, the continuous monitoring of pH, that can be shown on a display of the device object of the invention, could therefore - in case of a huge discrepancy with a nominal predefined value linear function of time - to the interruption of the treatment and therefore the repetition of the treatment (see above).

On the display are advantageously shown also some parameters among which there are the therapy time and the value of electric charge and/or of electric current delivered through the electrodes.

The data processing unit 110 furthermore controls a constant current generator 120 with a signal of generation of electric current, whose value is associated to the type of pathology of the treated tooth. The type of pathology of the treated tooth is manually set by the user through the action on the user interface device 140.

The constant current generator 120 comprises an output electrically directly connected with the first electrode 131.

The constant current generator is fed by means of a voltage generator 170 and has a secondary input of control to which is connected a current sensor 180, in turn controlled by an automatic activation/deactivation stage 190. The stage of automatic activation/deactivation 190 advantageously allows for interrupting the delivering of electric current in accordance to parameters of electric resistance found between the first and the second electrode 131, 132 calculated also thanks to the current integrator 200.

In detail, the interruption of delivering of electric current on the output of the constant current generator 120 is performed when, alternatively or in combination:
- the value of electric resistance found between the first and the second electrode 131, 132 is above a threshold value of electric resistance in advance stored within the memory 150; and/or
- the value of electric resistance changes in time with a temporal derivative whose value is above a predetermined value stored within the memory 150.

This allows advantageously of preventing forcing of the internal electric circuit of the device 100 object of the present invention in case of clear system malfunction or in case of voluntary or casual extraction of one of the two electrodes.

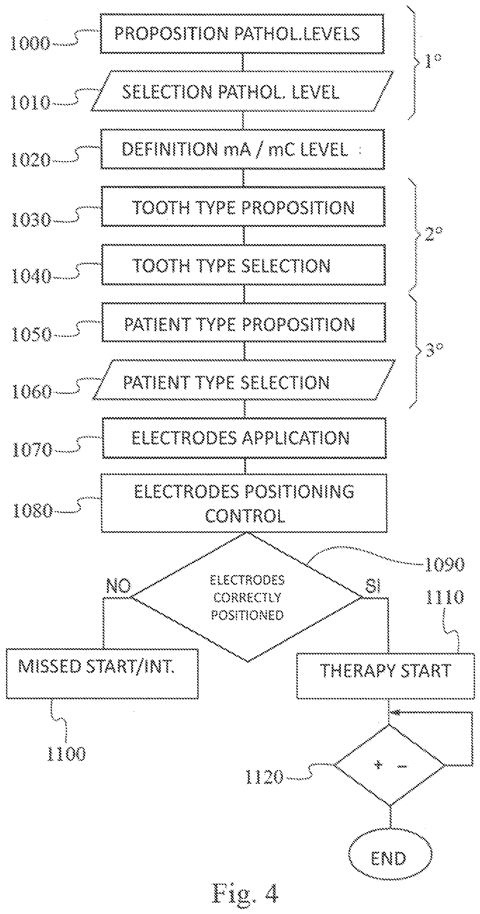

With reference to flow chart of figure 4, in use, the dentist firstly detects the level of seriousness of the pathology, framing it into one of the four levels assigned thereto, through the touch-sensitive monitor. Technically, in this phase, the data processing unit 110 loads from memory 150 the names of the four levels of pathology and sends them on said monitor 140 (block 1000).

The dentist then selects (block 1010) one of the four levels of seriousness of pathology according to its framing, by touching the touch-sensitive monitor 140 in correspondence of said level of pathology.

Therefore, the selection causes a transmission (block 1020) of a command between the touch-sensitive monitor 140 and the data processing unit 110; this last loads from memory 150 the parameters corresponding to the selected level of pathology - in detail the necessary electric current and the corresponding electric charge - and sends them with a signal of generation of electric current in such a way that the constant current generator 120 delivers an electric current towards the electrode with an intensity equal to that associated to said selected level of pathology.

Then the data processing unit 110 transmits (block 1030) on said touch-sensitive monitor 140 a signal that causes the projection of a menu of request of the number of channels to be treated. The dentist selects the number of channels to be treated (block 1040), and this number is temporarily stored in the data processing unit or into the memory for the amount of time which is necessary for executing the therapy. Then the data processing unit 110 causes the transmission (block 1050) on the touch-sensitive display 140 of a message of selection between two types of patient on said touch-sensitive monitor. Said two types of patient correspond to:
- normal patient; or
- sensitive patient.

According to the type of patient that the dentist selects by touching the touch-sensitive monitor 140 the data processing unit 110 recontrols the values of intensity of electric current provided to the current generator; in detail:
- if the type of selected patient is "normal", for the therapy will be used the value of current into the upper column of the interval of values of currents which are admissible for the determined level of pathology;
- if the type of selected patient is "sensitive", for the therapy will be used the value of current into the lower column of the interval of values of current which are admissible for the determined level of pathology.

This advantageously allows the dentist to have a higher easiness of therapy parameters preselection in accordance to the sensitivity of the patient, treating therefore patients also with a pain threshold significantly lower than the normality without requiring a manual modification of current values.

Then at first the electrode is applied in the tooth channel and the second electrode on the hand of the patient (block 1060).

The therapy begins when the dentist touches the touch-sensitive monitor 140 in correspondence of a virtual button of activation of the therapy, LI HASTO starting from a signal generated by the data processing unit; by pushing said button, the data processing unit commands the effective activation of the electric current generator. Said electric current flows through the needle-shaped body 131t positioned within the tooth of the patient and from this last until the second electrode 132, closing the circuit.

The data processing unit 110 begins a counting of time decreasing remaining for the therapy, deactivating the electric circuit in case said time is deleted. Said time counting (block 1120) is predetermined in accordance to the quantity of electric charge to provide depending on the pathology in advance selected by the dentist between the plurality of pathologies present in memory 150.

At the end of the counting, if the number of channels to treat is equal to 1, the therapy is ended. In contrast, if the number of channels to treat is higher than 1, then the data processing unit - according to said number of channels to treat, will repeat a plurality of times a command of transmission of an image of the modification of the tooth channel to treat, followed by a new projection of a virtual button for the activation of the therapy, this time on the channel adjacent to the preceding one. The transmission of said image takes place for a number of times equal to the number of tooth channels minus 1.

During all the cycle of sterilization of the radicular channel, the value of electric current provided is kept constant. Therefore, independently from the value of electric resistance encountered, with the obvious limits given by the electric tension generator.

During all the time of the therapy, the data processing unit 110 performs a systemic control of the electric resistance encountered at the passage of the electrodes. In particular (block 1090) this control is also performed before the start of the therapy itself, such as to allow for avoiding the delivering of the electric current if the electrodes are wrong positioned. Said control, also in this case, is a control based on the electric resistance.

During the therapy performed with the device proposed in the present invention, the dentist can force the delivering of the continuous electric current between the first and the second electrode 131, 132 up to a maximum value of 5mA, or up to a minimum value of 0,5mA, also therefore out of these intervals detected by the previously mentioned table, the maximum limit of 5mA is determined in such a way to ensure the safety of the patient by preventing electrocution events. In the same way, the duration of the therapy can be manually increased and the delivering of electric current can be manually interrupted by means of pushing the button of activation and deactivation on the handle 131p of the first electrode 131.

Although primarily the current is of continuous type, into the treatment of determined types of pathologies can be necessary the use of a pulsed electric current, of which the mean value anyway does not exceed the maximum of 5mA.

In case of pushing the button of activation on the handle 131p, from said button is sent to the data processing unit 110 a stop signal that provides for interrupting also the time counting of the duration of the therapy. Advantageously, this allows for keeping unaltered the effective duration of the therapy also in case wherein there are one or more than one interruptions.

It is to be claimed in this field that an adaptive procedure from the control system conforms itself in relation to the different sensitivity that the patient shows in the course of the treatment. It automatically brings to deliver electric charges for a period more or less long if the initial response is not received and the behavior is no more linear as previously evidenced.

It is hereinafter described the arrangement of the sensor unit for the diagnosis of the conditions of the tooth. The operation bases on a sensor which is configured for being placed on the tooth 200 of the patient in punctual contact with its specific zones. The sensor unit is capable of providing detectable signals showing the presence of particular substances like can be the hydrogen ion, the calcium ion, bacteria and bacterial metabolites. The sensor furthermore communicates the data to a section of the data processing unit 110, devoted to the management of the detected signal that is configured in such a way to interoperate both with the sensor unit and with the other units composing the system of sterilization using the ionic current. The data processing unit 110 processes the parameters which are detected and provides a derived signal which is indicative of the conditions of the tooth into the zone under monitoring. The processor is furthermore capable of transmitting the so derived signal to a memory and of potentially showing the trend of the detected measure on a specific device by using a set of diagnostic messages. Most of all, it is capable of interoperating with a program for the automatic control - resident in the data processing unit 110 that according to a specific algorithm, hereinafter specified, is capable of providing the correction to introduce in the treatment of sterilization - into the amount of charge delivered - that is being actuated respective to the tooth.

In fact, the sensor unit is capable of providing in real time a signal referring to the concentration of the already cited substances, while the data processing unit 110 and memory 150 can, from one side acquire the information from the sensor about the conditions of the tooth and at the same time intervene on the sterilization process using the ionic current, providing interactively a variation in time of the treatment and therefore into the amount of Coulomb used in the system of sterilization.

In addition, in another embodiment, the sensor unit is capable of operating in such a way to provide a further function of visualization of the tooth subject to treatment and a series of parameters indicative of the related integrity during the intervention. The type of diagnostic detection which is recalled by the datum provided by the sensor is indicative of the conditions of the tooth, in particular in the zones which are interested by the decay, of the person under examination, and is the base of the process of setting of the intervention by the device, that actuates the proceeding of sterilization using the ionic current to the scope of optimizing the recuperation of the tooth.

A typical sensor according to the present invention is structured in such in an elongated shape, preferably in a pointed form, the structure having an extremity with an handle suitable for being grasped by the user's fingers and a sensitive end suitable for being arranged in proximity of the tooth.

Another structuration of the sensor has the response of the minimal variation according to different fields of pH, with a fine sensitivity optimized for variations of pH in a pre-determined range of values.

The specific use of said device can comprise the measurement of other parameters like the concentration of the hydrogen ions in the saliva, the density of the
tartar, the concentration of hydrogen ions and bacteria contained in the oral cavity. The sensor, as described, cooperates with other types of sensors not operating directly with the tooth, for example can be used devices for detecting acidity, the amount of ions and the content of bacteria surrounding the actual tooth ( for example referring to the saliva extracted by the mouth by means of a capillary tube which is connected to an instrument for measuring and detecting or referring to the saliva which is drawn and dried in proximity of the tooth which is subjected to the treatment).

The pH probe properly solves the issue of the evaluation pf the grade of alkalization. The implemented algorithm provides for:
a- a preliminary off-line detection of pH value of the tooth in correspondence of a specific point of it;
b- a preliminary detection of the specific sensitivity of the caries to the treatment in progress.

According to said initial detection, according to the type of tooth that will be object of the treatment, as well as according to the entity of the caries as it is highlighted, is automatized the sending of a quantity of Coulomb q for an interval ΔTx, predetermined in a setup table, that considers the different parameters upon which the initialization is carried out. On the abscises of the diagram are reported times and therefore the quantity of supplied charge, whereas on the ordinates are reported the values of pH upon variation of time and therefore of supplied Coulombs. Each bundle of straight lines corresponds to a determined type of tooth that has an initial specific value of pH, and to the related incremental linearized treatment. It is clear that the sensitivity to the treatment depending on the type of caries, on pH or on other objective parameter factor, can be more or less compact and therefore more or less permeable to the ionic current treatment.

In relation to the particular type of tooth and to an initial evaluation of the tooth it is obtained a "permeability coefficient" of the caries to OH- ion according to the difference of values of pH1 and pH2 measured on a predetermined time interval. Said pH increase over the time unit in relation to a predetermined quantity of charge allows for defining a preset parameter upon which the control system manages the whole sterilization process.

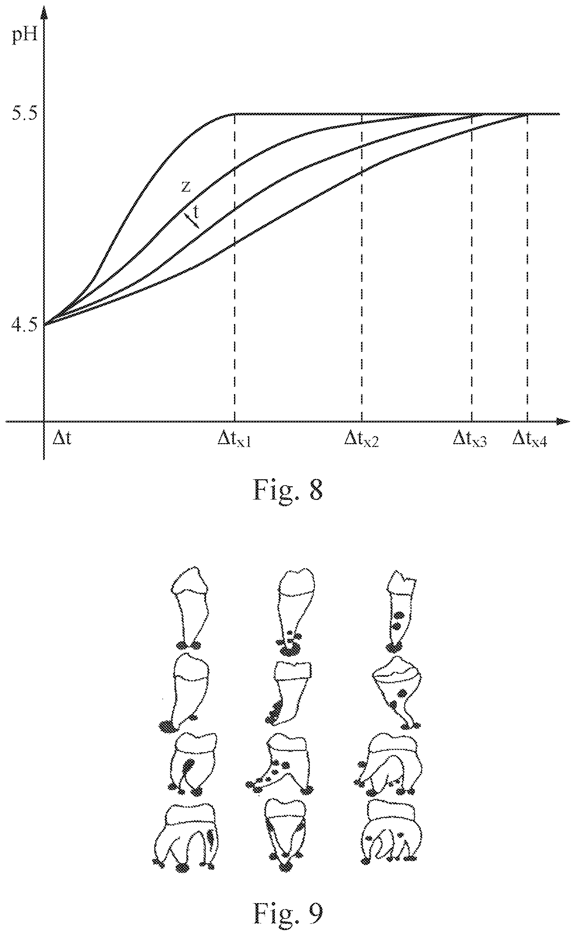

In fact, following said pre-positioning, it is activated a cycle ending when ΔTx period finishes wherein is constantly delivered the quantity of charge provided for the particular case during the treatment as it is shown in Fig. 8. Substantially, the increase trend of pH over time unit indicates the sensitivity to the treatment in relation to the type of caries, that further than pH, can be more or less compacted and therefore less permeable to the type of supplied charge.

More in particular, it is defined a preset phase wherein according to the type of tooth and to the initial pH of the tooth is sent a first constant quantity - test dose - of charge over a normalized Δt for each type of tooth.

As the Δt interval of prepositioning ends when the cycle 110 ends, is again carried out the detection of pH (and/or Ca++, and/or SO4--) that is present into the same identical area of the tooth wherein the previous detection was made and that corresponds to the area of treatment. Said double distanced detection of Δt period with the test ionization treatment, allows the machine to acquire a first information related to the response of the particular type of caries that is under treatment with respect to the ionic current. Typical values according to which said first test takes place respectively stop on the following values, reported by way of example, in Fig. 6 wherein is certainly detected the different increase trend of pHi in the two cases wherein the first starts from a low value and the second from an already high value. While in the previous solution it was generally defined a granuloma or an infection but it was not known the depth or the extent or how much it was compact and impermeable to OH- ion, in the present invention instead it is considered first of all the initial pH (it is evident that an initial pH equal to 4 is different from a pH equal to 5) and starting from the value it is considered the specific reactivity of the caries to the ionization treatment further than what is visually seen by the dentist. Above all, it is important that the initialization provides that preliminary it is detected the different type of response according to the pH and according to the type of solicitation firstly supplied.

Once it is defined the type of caries and the sensitivity of the infected area of the tooth to the treatment with ionic current the processor is capable of defining for each tooth in relation to the related pathology, the ΔTx interval (with x depending on the previously described preliminary evaluation) during which supplying current and continuously controlling the delivering of the current by cyclically ordering an adjustment relation between the detected pH and the quantity of Coulomb to be delivered. In fact, according to a linear interpolation process it is dynamically controlled at discrete time intervals the increase of pH in relation to the delivered quantity of Coulomb as it is highlighted from the cycle wherein with the control are positioned in relation the different input parameters with the delivered current flow. The control system provides for a substantially linear and directly proportional trend and between the quantity of released charge and the contextually measured value of pH that increases - as result of the neutralization reaction.

With the continuous line has been reported the trend for a simple caries, with the dotted line a spongy caries (with presence of air spaces between a cell and the other), with the dash-dot line a more compact caries.

It is fundamental that considering the real-time detection of the del pH - variations a sit could be in figure 7 for the curve indicated with Z, a type sequence can be reported that over a particular threshold of the pH the increase of it over time unit is not anymore the one that was estimated and therefore, fundamentally, the time originally expected, corresponding to ΔTxz, will not be anymore sufficient for reaching the desired result. Upon the occurrence of said trend in disagreement with the initial projections, the control system defines itself a translation on the working curve Y lower down to which is associated a reaching time of the target pH ΔTx3>ΔTx2.

In a further embodiment of the finding, significant deviations of the linearized trend provide that is proportionally lowered or increased the delivered current flow over time unit in relation, respectively, to the fact that the pH has increased more or lower than expected.

It is also evident that many parameters, further than pH, can be followed simultaneously to the end of obtaining an optimization of the process of detection and a more fine setting of the variations of delivered charge flow.

The advantages of the device 100 object of the present invention are clear in the light of the description that precedes. It allows for processing a control of a more easy type, that allows to the dentist or anyway to the doctor, a rapid selection of a particular predefined therapy according to preset parameters.

The device object of the present invention is furthermore constantly active in the search of potential wrong positioning of electrodes that could be dangerous for the effectiveness of the therapy or also being harmful for what concerns the health of the patient.

Even if throughout the present description explicit reference has been made to the teeth canalar sterilization, it is true as well that the device object of the present invention can be applied for example for sterilizing metal parts inside the body like for example prostheses.

A further application of the present invention is furthermore the sterilization for example of metal cans containing food.

## Claims

1. Device for disinfecting by means of ionic current, said device (100) comprising a principal body provided with of a data processing unit (110) suitable for feeding a constant current generator (120) with a signal of generation of an electric current fed to a couple of electrodes (131, 132) electrically correlated thereto, and comprising furthermore a user interface device (140) configured for allowing of selecting a determined pathology between a plurality of pathologies in advance stored within a memory (150) electrically connected with said data processing unit (110), wherein for each pathology of the said plurality of pathologies is in advance stored in said memory a interval of values of electric current and/or of electric charge and/or a specific value of electric current and/or of electric charge that said constant current generator (120) shall deliver during said therapy,
and wherein said couple of electrodes (131, 132) comprises a first electrode of an elongated type (131) suitable for being introduced or anyway put in proximity of an infected or inflamed portion of human body, said portion being a bone or tooth or respective cavities thereof, and a second electrode (132) suitable for being hold by an ill patient; said device being **characterized in that** within said memory (150) are stored, for each pathology of the said plurality of pathology, a first interval of values of electric current and a second interval of values of electric current and at least a first value of charge; said first interval and said second interval being alternatively selected by said data processing unit in accordance to a command of selection imposed by the user to said data processing unit.

2. Device according to claim 1, **characterized in that** said means of interface user (140) are configured for allowing of selecting one between a plurality of levels of sensitivity of the patient and for causing the transmission of said signal of generation of electric current to said constant current generator in accordance to said selection performed from said user, said transmission of said signal of generation of electric current being performed in relation to said selection command.

3. Device according to any of the preceding claims, **characterized in that** it comprises sensor means of current, measuring the electric current flowing between said first and second electrode (131, 132); said sensor means of current being interfaced with said data processing unit (150) per calculate a electric resistance to the passage of said current and interrupting said passage of electric current between said first and second electrode (131, 132) in case said electric resistance exceeds a threshold value in advance stored within said memory (140) and/or said value of electric resistance changes with a derivative exceeding a predetermined value; said data processing unit (150) continuously calculating said value of electric resistance.

4. Device according to any of the preceding claims, wherein said interval of values of electric current is comprised between 0,5mA and 5mA, and said value of charge is comprised between 100mC and 1000mC; and wherein for each pathology selected between said plurality of pathologies in advance stored said signal of generation of electric current and/or charge tracts a electric current and/or charge comprised in the interval associated to said selected pathology.

5. Device according to any of the preceding claims, **characterized in that** said first electrode (131) comprises a needle-shaped portion (131s) ending in correspondence to an own end with a portion which is tapered at the point; said first electrode (131) comprising furthermore an electrically isolated section, covered by a layer of electrically isolating material or paint of thickness smaller than the thickness of the section of said needle-shaped portion (131s).

6. Device according to any of the preceding claims, **characterized in that** said first electrode (131) comprises a needle-shaped portion (131s) ending in an end thereof with a rounded portion of a size greater respective to the section of said needle shaped portion (131s); said first electrode (131) comprising furthermore an electrically isolated section, covered by a layer of electrically isolating material or paint of thickness smaller than the thickness of the section of said needle-shaped portion (131s).

7. Device according to any of the preceding claims, **characterized in that** it comprises a needle shaped portion (131s) ending in a first extremity thereof with a double point suitable for introducing in a plurality of channels of said tooth.

8. Device according to any of the preceding claims, **characterized in that** said user interface device (140) configured for allowing to select a specific type of bone or tooth between a plurality of types of bone or tooth, and wherein said electric current and/or charge delivered by said constant current generator towards said couple of electrodes (131, 132) is delivered according to a signal of generation of electric current transmitted from said data processing unit (150) towards said constant current generator in accordance to said specific selected type of bone or tooth.

9. Device according to claim 8, wherein said type of tooth is selected in accordance to a number of channels of said tooth, and wherein said data processing unit (150) calculates a predetermined time of transmission of said electric current to said couple of electrodes (131, 132) for each of the channels of the selected tooth type, and is configured for performing a cycle of sending of an alert message on said user interface means in case the predetermined time for said therapy of said channel is ended, wherein said cycle is performed for a number of times equal to the number of channels of said tooth minus 1; said data processing unit (150) being configured for waiting a command of the user for sending the signal of generation of the electric current for treating the adjacent channel, up to moment where that cycle is arrested.

## Patentansprüche

1. Vorrichtung zur Desinfektion mittels ionischen Stroms, wobei die Vorrichtung (100) einen Hauptkörper umfasst, der mit einer Datenverarbeitungseinheit (110) versehen ist, die dazu geeignet ist, einem Generator von konstantem Strom (120) ein Signal bezüglich der Erzeugung einer elektrischen Stromzuführung zu einem Paar von dazu elektrisch korrelierten Elektroden (131, 132) zuzuführen, und ferner eine Benutzerschnittstellenvorrichtung (140) umfasst, die ausgestaltet ist, um die Wahl einer bestimmten Krankheit aus einer Vielzahl von Krankheiten zu ermöglichen, die zuvor in einem Speicher (150) abgelegt wurden, der mit der Datenverarbeitungseinheit (110) elektrisch verbunden ist, wobei ein Wertebereich des elektrischen Stroms und/oder der elektrischen Ladung und/oder ein spezifischer Wert des elektrischen Stroms und/oder der elektrischen Ladung, den der Generator von konstantem Strom (120) bei der Therapie zuführen sollte, für jede Krankheit aus der Vielzahl von Krankheiten zuvor im Speicher abgelegt wurde,
und wobei das Paar von Elektroden (131, 132) eine erste Elektrode vom länglichen Typ (131), die dazu geeignet ist, in einem oder auf jeden Fall in der Nähe eines infizierten oder entzündeten Abschnitts des menschlichen Körpers eingeführt oder gesetzt zu werden, wobei der Abschnitt ein Knochen oder Zahn oder jeweilige Hohlräume davon ist, und eine zweite Elektrode (132) umfasst, die dazu geeignet ist, von einem kranken Patienten gehalten zu werden; wobei die Vorrichtung **dadurch gekennzeichnet ist, dass** ein erster Wertebereich des elektrischen Stroms und ein zweiter Wertebereich des elektrischen Stroms und zumindest ein erster Wert der Ladung für jede Krankheit aus der Vielzahl von Krankheiten im Speicher (150) abgelegt sind, wobei der erste Bereich und der zweite Bereich alternativ durch die Datenverarbeitungseinheit gemäß einem Wahlbefehl ausgewählt werden, der vom Benutzer der Datenverarbeitungseinheit aufgeteilt wurde.

2. Vorrichtung nach Anspruch 1, **dadurch gekennzeichnet, dass** die Mittel der Benutzerschnittstelle (140) ausgestaltet sind, um die Wahl eines aus einer Vielzahl von Empfindlichkeitsniveaus des Patienten zu ermöglichen und die Übergabe des Signals bezüglich der Erzeugung des elektrischen Stroms zum Generator von konstantem Strom gemäß dieser vom Benutzer durchgeführten Wahl zu verursachen, wobei die Übergabe des Signals bezüglich der Erzeugung des elektrischen Stroms in Bezug auf dem Wahlbefehl durchgeführt wird.

3. Vorrichtung nach einem der vorhergehenden Ansprüche, **dadurch gekennzeichnet, dass** sie Sensormittel des Stroms umfasst, die den elektrischen Strom messen, der zwischen der ersten und der zweiten Elektroden (131, 132) strömt; wobei die Sensormittel des Stroms mit der Datenverarbeitungseinheit (150) verknüpft sind, um einen elektrischen Widerstand zum Durchgang des Stroms zu berechnen und den Durchgang von elektrischem Strom zwischen der ersten und der zweiten Elektroden (131, 132) zu unterbrechen, falls der elektrische Widerstand einen Schwellenwert überschreitet, der zuvor im Speicher (140) abgelegt wurde, und/oder der Wert des elektrischen Widerstands mit einer Ableitung variiert, die einen vorgegebenen Wert überschreitet; wobei die Datenverarbeitungseinheit (150) den Wert des elektrischen Widerstands kontinuierlich berechnet.

4. Vorrichtung nach einem der vorhergehenden Ansprüche, wobei der Wertebereich des elektrischen Stroms zwischen 0,5 mA und 5 mA liegt, und der Ladungswert zwischen 100 mC und 1000 mC liegt; und wobei das Signal bezüglich der Erzeugung des elektrischen Stroms und/oder der elektrischen Ladung für jede aus der Vielzahl von zuvor gespeicherten Krankheiten ausgewählte Krankheit einen elektrischen Strom und/oder eine elektrische Ladung ableitet, der/die im der ausgewählten Krankheit zugeordneten Bereich umfasst wird.

5. Vorrichtung nach einem der vorhergehenden Ansprüche, **dadurch gekennzeichnet, dass** die erste Elektrode (131) einen nadelförmigen Abschnitt (131s) umfasst, der an einem eigenen Ende ausläuft, das sich spitz verjüngt; wobei die erste Elektrode (131) ferner einen elektrisch isolierten Querschnitt umfasst, der durch eine Schicht aus elektrisch isolierendem Material oder Lack mit einer geringeren Dicke als der Dicke des Querschnitts des nadelförmigen Abschnitts bedeckt wird (131s).

6. Vorrichtung nach einem der vorhergehenden Ansprüche, **dadurch gekennzeichnet, dass** die erste Elektrode (131) einen nadelförmigen Abschnitt (131s) umfasst, der gekrümmt und größer dimensioniert als der Querschnitt des nadelförmigen Abschnitts (131s) an einem eigenen Ende ausläuft, das sich spitz verjüngt; wobei die erste Elektrode (131) ferner einen elektrisch isolierten Querschnitt umfasst, der durch eine Schicht aus elektrisch isolierendem Material oder Lack mit einer geringeren Dicke als der Dicke des Querschnitts des nadelförmigen Abschnitts bedeckt wird (131s).

7. Vorrichtung nach einem der vorhergehenden Ansprüche, **dadurch gekennzeichnet, dass** sie einen nadelförmigen Abschnitt (131s) umfasst, der in einem ersten Ende davon mit einer Doppelspitze ausläuft, die dazu geeignet ist, eine Vielzahl von Zahnkanälen einzuführen.

8. Vorrichtung nach einem der vorhergehenden Ansprüche, **dadurch gekennzeichnet, dass** die Benutzerschnittstellenvorrichtung (140) ausgestaltet ist, um die Wahl einer spezifischen Knochen- oder Zahnart aus einer Vielzahl von Knochen- oder Zahnarten zu ermöglichen, und wobei der elektrische Strom und/oder die elektrische Ladung durch den Generator von konstantem Strom zum Paar von Elektroden (131, 132) gemäß einem Signal bezüglich der Erzeugung des elektrischen Stroms zugeführt werden, das durch die Datenverarbeitungseinheit (150) zum Generator von konstantem Strom gemäß der spezifischen ausgewählten Knochen- oder Zahnart übergeben wird.

9. Vorrichtung nach Anspruch 8, wobei die Zahnart gemäß einigen Kanälen des Zahns ausgewählt wird, und wobei die Datenverarbeitungseinheit (150) eine vorbestimmte Übergabezeit des elektrischen Stroms zum Paar von Elektroden (131, 132) für jedes der Kanäle der zweiten Zahnart berechnet, und ausgestaltet ist, um einen Zyklus durchzuführen, in dem eine Warnmeldung auf die Mittel der Benutzerschnittstelle gesendet wird, falls die vorbestimmte Zeit für die Therapie des Kanals beendet wird, wobei der Zyklus soviel mal durchgeführt wird, wie die Anzahl der Zahnkanäle minus 1; wobei die Datenverarbeitungseinheit (150) ausgestaltet ist, um den Befehl des Benutzer abzuwarten, um das Signal bezüglich der Erzeugung des elektrischen Stroms zu senden, um den begrenzenden Kanal zu behandeln, bis der Zyklus aussetzt.

## Revendications

1. Dispositif pour désinfection au moyen de courant ionique, ledit dispositif (100) comprenant un corps principal pourvu d'une unité de traitement de données (110) permettant d'alimenter un générateur de courant constant (120) avec un signal de génération d'un courant électrique alimenté à un couple d'électrodes (131, 132) électriquement corrélé au même, et comprenant de plus un dispositif d'interface utilisateur (140) configuré pour permettre la sélection d'une pathologie déterminé entre une pluralité de pathologies précédemment stockées dans une mémoire (150) reliée électriquement à ladite unité de traitement de données (110), dans laquelle, pour chacune pathologie de ladite pluralité de pathologies, un intervalle de valeurs de courant électrique et/ou de charge électrique et/ou une valeur spécifique de courant électrique et/ou de charge électrique, que ledit générateur de courant constant (120) doit envoyer pendant ladite thérapie, sont précédemment stockées dans ladite mémoire,
et dans lequel ledit couple d'électrodes (131, 132) comprend une première électrode du type allongé (131), permettant d'être introduite ou, du moins, mise à proximité d'une partie infectée ou enflammée du corps humain, ladite partie étant un os ou un dent ou des respectives cavités des mêmes, et une seconde électrode (132), permettant d'être soutenue par un patient malade; ledit dispositif étant **caractérisé en ce que** dans ladite mémoire (150) sont stockées, pour chacune pathologie de ladite pluralité de pathologies, un premier intervalle de valeurs de courant électrique et un second intervalle de valeurs de courant électrique et au moins une première valeur de charge; ledit premier intervalle et ledit second intervalle étant alternativement sélectionnés par ladite unité de traitement de données selon un ordre de sélection imposé par l'utilisateur à ladite unité de traitement de données.

2. Dispositif selon la revendication 1, **caractérisé en ce que** lesdits moyens d'interface utilisateur (140) sont configurés pour permettre la sélection d'une entre une pluralité de niveaux de sensibilité du patient et pour provoquer la transmission dudit signal de génération de courant électrique audit générateur de courant constant selon ladite sélection réalisée par ledit utilisateur, ladite transmission dudit signal de génération de courant électrique étant réalisée par rapport audit ordre de sélection.

3. Dispositif selon l'une quelconque des revendications précédentes, **caractérisé en ce qu'**il comprend moyens de détection de courant, qui mesurent le courant électrique qui passe entre ladite première et seconde électrode (131, 132); lesdits moyens de détection de courant étant reliés avec ladite unité de traitement de données (150) pour calculer une résistance électrique au passage dudit courant et interrompre ledit passage de courant électrique entre ladite première et seconde électrode (131, 132) si ladite résistance électrique dépasse une valeur seuil précédemment stockée dans ladite mémoire (140) et/ou ladite valeur de résistance électrique varie avec une dérivée qui dépasse une valeur prédéterminée; ladite unité de traitement de données (150) calculant de façon continuelle ladite valeur de résistance électrique.

4. Dispositif selon l'une quelconque des revendications précédentes, dans lequel ledit intervalle de valeurs de courant électrique est compris entre 0,5 mA et 5 mA, et ladite valeur de charge est comprise entre 100 mC et 1000 mC; et dans lequel, pour chacune pathologie sélectionné entre ladite pluralité de pathologies précédemment stockées, ledit signal de génération de courant électrique et/ou charge sort une courant électrique et/ou une charge électrique compris/comprise dans l'intervalle associé à ladite pathologie sélectionnée.

5. Dispositif selon l'une quelconque des revendications précédentes, **caractérisé en ce que** ladite première électrode (131) comprend une partie en forme d'aiguille (131s) qui termine en correspondance d'une de ses extrémités avec une partie qui est en entonnoir à pointe; ladite première électrode (131) comprenant de plus une section isolée électriquement, couverte par une couche de matériau électriquement isolé ou vernis, avec une épaisseur inférieure à l'épaisseur de la section de ladite partie en forme d'aiguille (131s).

6. Dispositif selon l'une quelconque des revendications précédentes, **caractérisé en ce que** ladite première électrode (131) comprend une partie en forme d'aiguille (131s) qui termine en correspondance d'une de ses extrémités avec une partie arrondie avec une dimension supérieure par rapport à la section de ladite partie en forme d'aiguille (131s); ladite première électrode (131) comprenant de plus une section isolée électriquement, couverte par une couche de matériau électriquement isolé ou vernis, avec une épaisseur inférieure à l'épaisseur de la section de ladite partie en forme d'aiguille (131s).

7. Dispositif selon l'une quelconque des revendications précédentes, **caractérisé en ce qu'**il comprend une partie en forme d'aiguille (131s) qui termine dans une de ses extrémités avec une pointe double permettant d'être introduite dans une pluralité de canaux dudit dent.

8. Dispositif selon l'une quelconque des revendications précédentes, **caractérisé en ce que** ledit dispositif d'interface utilisateur (140) est configuré pour permettre la sélection d'un type spécifique d'os ou de dent entre une pluralité de types d'os et de dent, et dans lequel ledit courant électrique et/ou ladite charge électrique est fournie/fourni par ledit générateur de courant constant vers ledit couple d'électrodes (131, 132) selon un signal de génération de courant électrique transmis de ladite unité de traitement de données (150) vers ledit générateur de courant constant selon le type spécifique sélectionné d'os ou de dent.

9. Dispositif selon la revendication 8, dans lequel ledit type de dent est sélectionné selon un certain nombre de canaux dudit dent, et dans lequel ladite unité de traitement de données (150) calcule une période prédéterminée de transmission dudit courant électrique vers ledit couple d'électrodes (131, 132) pour chacun des canaux du type de dent sélectionné, et est configuré pour réaliser un cycle d'envoi d'un message d'alerte sur lesdits moyens d'interfaces utilisateur si la période prédéterminée pour ladite thérapie dudit canal est terminée, dans lequel ledit cycle est réalisé pour un nombre de fois égal au nombre de canaux dudit dent moins 1; ladite unité de traitement de données (150) étant configurée pour attendre un ordre de l'utilisateur pour envoyer le signal de génération du courant électrique pour traiter le canal adjacent, jusqu'à ce que ledit cycle s'arrête.
